# EUROPÄISCHE PATENTANMELDUNG

(11) **EP 4 574 238 A1**
(43) Veröffentlichungstag der Anmeldung: **25.06.2025**
(21) Anmeldenummer: 24214292.5
(22) Anmeldetag: 20.11.2024
(51) Int. Cl.: B01D 53/04, B01D 39/00, B01D 46/00, B01J 20/06, B01J 20/20, B01J 20/28, B01J 20/30, B01D 53/02

(54) **FILTEREINHEIT**

(30) Priorität: 22.11.2023 DE 102023132613
(71) Anmelder: Westa-Holding GmbH & Co. KG, 33334 Gütersloh (DE)
(72) Erfinder: Westerbarkey, Peter, 33332 Gütersloh (DE)
(74) Vertreter: DTS Patent- und Rechtsanwälte PartmbB

(57) **Zusammenfassung**

Filtereinheit (1, 1'), insbesondere zur Feinfiltration von Fluiden und Gasen, aufweisend mindestens eine Einlassseite (2), mindestens eine Auslassseite (5) und einen Adsorptionskörper (4) in Gestalt eines Formkörpers, der aus einem Adsorptionsmittel (4.1), insbesondere aus Körner aus Aktivkohle, einem Bindemittel (4.2) und einer Zirconium-Verbindung (4.3) besteht, wobei das Adsorptionsmittel (4.1) mit dem Bindemittel (4.2) beschichtet ist und ferner die Zirconium-Verbindung (4.3) im Formkörper bindet.

## Beschreibung

Die Erfindung betrifft eine Filtereinheit - insbesondere zur Feinfiltration von Fluiden und Gasen - und ein Verfahren zur Herstellung der Filtereinheit.

Aus dem Stand der Technik sind eine Vielzahl von Filtereinheiten zur Feinfiltration von Fluiden und Gasen sowie Verfahren zu deren Herstellung bekannt, wie bspw. Aktivkohlefilter.

Bei Aktivkohlefiltern wird als Adsorptionsmittel Aktivkohle verwendet. Die Aktivkohle kann lose in den Filter eingebracht werden oder, um eine sich selbsttragende Struktur bzw. Formgebung zu erreichen, wird die Aktivkohle mittels Bindemittel in der Filtereinheit fixiert.

In einem wertvollen Beitrag aus dem Stand der Technik ist aus der DE 0 554 223 A1 ein Aktivkohlefilter und ein entsprechendes Verfahren zu dessen Herstellung beschrieben.

Mit den aus dem Stand der Technik bekannten Aktivkohlefilter lassen sich u.a. Chlor, organische Schadstoffe, Farb-, Geschmacks- und Geruchsstoffe aus Gasen, Dämpfen und Flüssigkeiten neutralisieren.

Nachteilig an den Filtereinheiten ist, dass viele Stoffe nicht oder nicht ausreichend filtrierbar sind. Schlecht filtrierbare Stoffe sind bspw. Nitrate. Mit den bekannten Filtereinheiten ist es folglich kaum möglich, diese Stoffe ausreichend zu adsorbieren.

Daraus ergibt sich die Aufgabe, eine Filtereinheit vorzusehen, mit der die Nachteile des Stands der Technik überwunden werden und den Adsorptionsgrad der Filtereinheit zu verbessern.

Die Aufgabe wird durch eine Vorrichtung und ein Verfahren nach den unabhängigen Ansprüchen 1 und 10 gelöst. Vorteilhafte Weiterbildungen sind in den Unteransprüchen definiert.

Die Aufgabe wird gelöst durch eine Filtereinheit, insbesondere zur Feinfiltration von Fluiden und Gasen, aufweisend mindestens eine Einlassseite, mindestens eine Filtrat-Auslassseite und einen Adsorptionskörper in Gestalt eines Formkörpers, der aus einem Adsorptionsmittel, insbesondere aus Körnern einer Aktivkohle, einem Bindemittel und einer Zirconium-Verbindung besteht, wobei das Adsorptionsmittel mit dem Bindemittel beschichtet ist und ferner die Zirconium-Verbindung im Formkörper bindet.

Die Filtration ist ein Verfahren zur Trennung oder Reinigung eines Mediums - einer Suspension oder eines Aerosols.

Eine filtrierte Flüssigkeit wird Filtrat genannt. Für ein filtriertes Gas wird die Bezeichnung Reingas verwendet.

Adsorptionsmittel sind bevorzugt wasserunlösliche poröse Materialien, die aufgrund ihrer großen Oberfläche Wasser oder andere Moleküle durch physikalische Kräfte an sich binden. Die Mittel werden zur Adsorption eingesetzt. Bevorzugte Adsorptionsmittel sind Bentonit, Aluminiumoxid, Kieselgel, Zeolithe und besonders bevorzugt Aktivkohle.

Aktivkohle, ist eine offenporige feinkörnige Kohle mit einer großen inneren Oberfläche, die als Adsorptionsmittel unter anderem in der Wasser- und Abwasserbehandlung sowie Lüftungs- und Klimatechnik eingesetzt wird. Aktivkohle besteht überwiegend aus Kohlenstoff (meist > 90 %) mit hochporöser Struktur. Die Poren sind untereinander verbunden (offenporig). Die innere Oberfläche beträgt bevorzugt zwischen 300 und 2000 m²/g Kohle. Die Dichte von Aktivkohle liegt im Bereich von 200 bis 600 kg/m³.

Für die Porengröße und die Porengrößenverteilung sind drei Größenordnungen von Bedeutung. Man unterscheidet zwischen Mikroporen (< 2 nm), Mesoporen (2 mm bis 50 nm) und Makroporen (> 50 nm).

Die Makroporen sind dabei die Hauptzugangswege für die Gase oder Flüssigkeiten in das Innere der Kohlen. Sie haben für die Adsorption aber praktisch keine nennenswerte Bedeutung. Der überwiegende Anteil der Adsorption erfolgt an dem Kohlenstoffmaterial, das an der Oberfläche der Mikroporen angeordnet ist. Dieser Bereich ist damit die eigentlich wirksame Oberfläche und bestimmt die Adsorptionseigenschaften der Kohle.

Bevorzugt werden die Körner aus Aktivkohle mit dem thermoplastischen Bindemittel unter Temperatureinwirkung beschichtet. Den mit Bindemitteln beschichteten Körnern aus Aktivkohle wird die Zirconium-Verbindung zugegeben. Das entstehende Gemisch wird komprimiert und anschließend über den plastischen Bereich des Bindemittels hinaus (unter Austreibung der flüchtigen Anteile) erhitzt, so dass die Zirconium-Verbindung vom Bindemittel zusätzlich im Adsorptionskörper am Bindemittel fixiert/gebunden wird. Dabei wird das Bindemittel derart aktiviert, dass es ebenfalls als Adsorptionsmittel wirkt. Die Aktivierung des Bindemittels erfolgt durch Erhitzung (unter Austreibung der flüchtigen Anteile) über den plastischen Bereich hinaus, dass das Bindemittel ebenfalls als Adsorptionsmittel wirkt.

Der so gebildeten Formkörper weist nach Abkühlung Mikroporen im Adsorptionsmittel, im Bindemittel und in der Zirconium-Verbindung auf, die alle wesentlich zur Adsorption beitragen.

Überraschenderweise bewirkt die Beimengung einer Zirconium-Verbindung im Gemisch aus Aktivkohle und Bindemittel, dass sich der Adsorptionsgrad der Filtereinheit bei der Adsorption zum Einen deutlich verbessert und zum Anderen Nitrate besser adsorbierbar sind, wobei ein Nitrat-Rückgang von mehr als 3% gegenüber herkömmlichen Aktivkohlefiltern gemessen werden konnte.

Bevorzugt ist die Zirconium-Verbindung eine Zirconiumchlorid-, eine Zirconiumsilicat-, eine Zirconiumoxid-, eine Zirconiumdioxid-, eine Zirconiumhydrid-, eine Zirconiumsulfat-, eine Zirconium-organische Verbindung und/oder eine Zirconiumlegierung.

Die Zirconium-Verbindung liegt (vor der Erhitzung über dem plastischen Bereich des Bindemittels hinaus) bevorzugt als Pulver oder als körniges Material vor.

Bevorzugt liegt die Zirconiumchlorid-, und/oder die Zirconiumsilicat-Verbindung in einem Bereich von 7,5 bis 8,4 Vol.-% des Adsorptionskörpers.

Bevorzugt liegt die Zirconiumoxid-, die Zirconiumdioxid-, und/oder die Zirconiumhydrid-Verbindung in einem Bereich von 7,5 bis 9,9 Vol.-% des Adsorptionskörpers.

Bevorzugt liegt die Zirconiumsulfat-, die Zirconium-organische Verbindung und/oder die Zirconiumlegierung in einem Bereich von 8,1 bis 8,8 Vol.-% des Adsorptionskörpers.

Bevorzugt beträgt der Volumenanteil der Zirconium-Verbindung ein Viertel bis ein Drittel des Volumenanteils des Bindemittels.

Bevorzugt macht das Bindemittel höchstens 30 Vol.-% des Adsorptionskörpers aus. Besonders bevorzugt ist ein Bindemittelanteil von 28 Vol.-%.

Bei einem Bindemittelanteil von höchstens 28 Vol.-%, hat sich in Versuchen gezeigt, dass diese Menge für die Beschichtung des Adsorptionsmittels ausreichend ist, die Adsorptionsfähigkeit des Adsorptionsmittels nicht zu beeinflussen, und gleichermaßen zu gewährleisten, die Zirconium-Verbindung noch ausreichend im Formkörper zu binden.

Bei einem Bindemittelanteil von 28 Vol.-%, bei 8 Vol.-% Anteil einer Zirconium-Verbindung und bei einem 64 Vol.-% Anteil des Adsorptionsmittels hat sich in Versuchen ferner gezeigt, dass sich gegenüber den bekannten Aktivkohlefiltern bessere Adsorptionsraten ergeben.

Ferner hat sich auch gezeigt, dass sich mittels dieser konkreten Zusammensetzung eine schnellere und wirkungsvollere Adsorption erzielen lässt, wobei ein Nitrat-Rückgang von mehr als 3% gegenüber herkömmlichen Aktivkohlefiltern gemessen werden konnte.

Bevorzugt ist das Bindemittel ein hochmolekularer thermoplastischer Kunststoff.

Hochmolekulare Kunststoffe sind Kunststoffe aus Makromolekülen, die aus sehr vielen, bis zu mehreren hunderttausend, gleichen oder unterschiedlichen Bausteinen (Atome oder Atomgruppen) bestehen und damit eine relativ große Molekülmasse aufweisen.

Thermoplaste sind Kunststoffe, die sich in einem bestimmten Temperaturbereich verformen lassen. Dieser Vorgang ist reversibel, d.h. er kann durch Abkühlung und Wiedererwärmung bis in den schmelzflüssigen Zustand beliebig oft wiederholt werden, solange nicht durch Überhitzung die sogenannte thermische Zersetzung des Materials einsetzt.

Thermoplastische Kunststoffe sind bevorzugt Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC).

Besonders bevorzugt ist das Bindemittel Polyethylen.

Bevorzugt weist die Struktur des Bindemittels eine Körnigkeit auf, welche Makroporen zwischen den Körnern des Adsorptionsmittels und der Zirconium-Verbindung freilässt.

Wie oben erwähnt dienen die Makroporen, als Hauptzugangswege für die Gase oder Flüssigkeiten. Das Bindemittel selbst weist auch eine Vielzahl von Mikroporen, die zur Adsorption dienen, auf.

Bevorzugt ist der Adsorptionskörper von einer einlassseitigen und einer auslassseitig anschließenden, porösen Wand begrenzt, wobei die Wände als mechanisches Feinfilter ausgebildet sind und die Wandstärken der Wände jeweils im Bereich von 5% bis 10% der Stärke des Adsorptionskörpers liegen.

Die Filtereinheit kann wirksam und wiederholt rückgespült werden, um den Adsorptionskörper vor mechanischer Verstopfung zu bewahren. Die anliegenden Wände und geben dem Adsorptionskörper zusätzlichen mechanischen Halt bei Druckbeanspruchungen, wodurch Beschädigungen oder Rissbildung im Formkörper verhindert werden. Ferner wird eine gleichmäßig verteilte Filtrat-Strömung gewährleistet.

Bevorzugt ist der Adsorptionskörper überwiegend zylindrisch und mit stirnseitig anschließenden Wänden von einem rohrförmigen Gehäuse umfasst oder der Adsorptionskörper weist eine hohle zylindrische Gestalt auf, wobei die Wände außen und innen jeweils eine Mantelfläche bilden.

Es hat sich gezeigt, dass bei der Dimensionierung der Wände mit einer Stärke im Bereich von 5% bis 10% des Adsorptionskörpers zur Optimierung der mechanischen Feinfiltration bereits an der einlassseitigen Wand beitragen. Eine Vielzahl von adsorbieren Stoffen lassen sich sofort an der einlassseitigen Wand filtern.

Es hat sich gezeigt, dass die Wand bevorzugt mindestens eine Stärke von 1,0 cm aufweisen muss, um überhaupt zur Optimierung der mechanischen Feinfiltration bereits an der einlassseitigen Wand beitragen zu können. Erst dann sind sinnvolle Ergebnisse zu erhalten.

Bevorzugt weist mindestens die einlassseitige Wand einen Sinterkörper auf, wobei der Sinterkörper aus mikroporösen Granulat-Körnern eines thermoplastischen Kunststoffes, aus einem Adsorptionsmittel aus Körnern von Aktivkohle und einer Zirconium-Verbindung besteht.

Ferner wird die Aufgabe gelöst durch ein Verfahren zur Herstellung einer Filtereinheit in Gestalt eines gas- und flüssigkeitsdurchlässigen, adsorptiven Formkörpers, wobei ein Adsorptionsmittel, insbesondere Körner aus Aktivkohle, mit einem thermoplastischen Bindemittel beschichtet werden, die Körner mit einer Zirconium-Verbindung homogen gemischt werden, das dabei entstehende Gemisch in eine Form eingebracht wird und in dieser komprimiert wird, das Gemisch in der Form bei Verhinderung von Luftzufuhr auf eine Temperatur wesentlich über den plastischen Bereich des Bindemittels hinaus erhitzt wird, die Zirconium-Verbindung im Gemisch gebunden wird und der so gebildeten Formkörper nach Abkühlung Mikroporen im Adsorptionsmittel, im Bindemittel und in der Zirconium-Verbindung aufweist.

Der Beschichtungsvorgang läuft dabei bevorzugt unter Temperatureinwirkung ab, wobei die Körner aus Aktivkohle, mit dem thermoplastischen Bindemittel beschichtet werden. Die Temperatur wird so gewählt, dass eine Beschichtung des Adsorptionsmittels gerade ermöglicht wird. Bevorzugt liegt die Temperatur zwischen 100°C und 180°C, besonders bevorzugt bei 150°C.

Dies hat den Vorteil, dass sich die Körner während des Verdichtungsvorgangs besser verdichten lassen, da die Gleitfähigkeit der Körner infolge der Temperatureinwirkung verbessert ist, ohne die Porosität des Adsorptionsmittels nennenswert einzuschränken.

Den mit Bindemitteln beschichteten Körnern aus Aktivkohle wird die Zirconium-Verbindung zugegeben, so dass eine homogene Verteilung im Gemisch gewährleistet ist. Das entstehende Gemisch wird komprimiert und anschließend weit über den plastischen Bereich des Bindemittels hinaus (unter Austreibung der flüchtigen Anteile) erhitzt.

Durch das Erhitzen über den plastischen Bereich des Bindemittels hinaus wird die Zirconium-Verbindung vom Bindemittel zusätzlich im Adsorptionskörper am Bindemittel fixiert/gebunden. Dabei wird das Bindemittel derart aktiviert, das es ebenfalls als Adsorptionsmittel wirkt.

Der so gebildeten Formkörper weist nach Abkühlung Mikroporen im Adsorptionsmittel, im Bindemittel und in der Zirconium-Verbindung auf, die alle wesentlich zur Adsorption beitragen.

Die Beimengung einer Zirconium-Verbindung im Gemisch aus Aktivkohle und Bindemittel bewirkt überraschenderweise, dass der Adsorptionsgrad der Filtereinheit bei der Adsorption zum Einen deutlich verbessert wird und die Adsorption schneller ablaufen kann. Zum Anderen sind Nitrate besser adsorbierbar, wobei ein Nitrat-Rückgang von mehr als 3% gegenüber herkömmlichen Aktivkohlefiltern gemessen werden konnte.

Bevorzugt beträgt die Temperatur beim Erhitzen des komprimierten Gemisches zwischen 255°C und 295°C und wird zwischen 35 Minuten und 45 Minuten konstant gehalten.

Bevorzugt wird der gebildete Formkörper im flüssigen oder gasförmigen Medium abgeschreckt.

Abschrecken ist ein Schritt der Wärmebehandlung von Materialien. Dabei wird der zuvor auf eine hohe Temperatur gebrachte Formkörper in Wasser, Öl oder durch Anblasen mit Luft schlagartig abgekühlt, so dass sich eine bestimmte Kristallstruktur ausbilden kann, die den Formkörper hart aber auch spröde macht.

Infolge des Abschreckens wird, wie gerade erwähnt, nicht nur die Härte und damit ein Beitrag zur selbsttragenden Struktur des Formkörpers erreicht, es kommt auch zu einer Erhöhung der Porosität und damit zu einer Zunahme von Mikro- und Makroporen, wodurch die Adsorptionsfähigkeit zusätzlich erhöht wird.

Bevorzugt wird der gebildeten Formkörper während des Abschreckens einer Temperatur im Bereich von -185°C bis -125°C ausgesetzt wird.

Bevorzugt dauert der Abschreckvorgang 20 Sekunden bis 180 Sekunden.

In Versuchen hat sich gezeigt, dass beim Abschrecken bei einer Temperatur von ca. -135°C und einer Dauer des Abschreckvorgangs von ca. 40 Sekunden der Adsorptionsgrad des Formkörpers deutlich verbessert wurde, so dass bei Nitraten ein Nitrat-Rückgang von mehr als 3% gegenüber herkömmlichen Aktivkohlefiltern gemessen werden konnte.

Im Folgenden werden weitere Ausgestaltungen der Erfindung beispielhaft anhand der beigefügten Zeichnungen erläutert.

Hierbei zeigen:
Figur 1 schematisch einen Querschnitt durch einen Feinfilter aus dem Stand der Technik.
Figur 2 schematisch einen Querschnitt durch eine alternative Ausführungsform eines Feinfilters aus Figur 1 aus dem Stand der Technik.
Figur 3 schematisch einen Schnitt durch ein Detail der Zusammensetzung bzw. des Aufbaus eines erfindungsgemäßen Adsorptionskörpers.

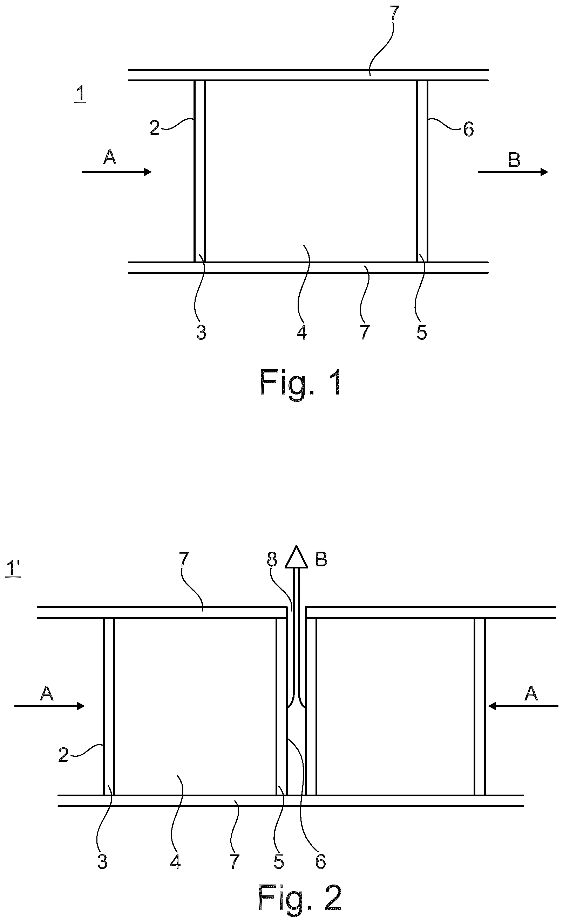

Figur 1 zeigt schematisch einen Querschnitt durch einen zylindrischen Feinfilter 1. Der Feinfilter 1 weist in Strömungsrichtung des Liquids eine lotrechte Einlassseite 2, eine Wand 3 an der Einlassseite 2, einen Adsorptionskörper 4, eine Wand 5 an einer Filtrat-Auslassseite 6 und die Filtrat-Auslassseite 6 selbst auf. Die einzelnen Komponenten der Filtereinheit 1 sind in einem rohrförmigen Gehäuse 7 angeordnet.

Dabei tritt das Liquid/Gas an der Einlassseite 2 des Feinfilters 1 ein (Pfeilrichtung A), durchströmt die Wand 3, den Adsorptionskörper 4, die Wand 5 und verlässt den Feinfilter als Filtrat/Reingas an der Filtrat-Auslassseite 6 (Pfeilrichtung B).

Die Wände 3 und 5 sind als mechanisches Feinfilter ausgebildet. Die Filtereinheit kann wirksam und wiederholt rückgespült werden, um den Adsorptionskörper vor mechanischer Verstopfung zu bewahren. Die anliegenden Wände 3 und 5 geben dem Adsorptionskörper zusätzlichen mechanischen Halt bei Druckbeanspruchungen, wodurch Beschädigungen oder Rissbildung im Formkörper verhindert werden. Ferner wird eine gleichmäßig verteilte Filtrat-Strömung gewährleistet.

Figur 2 zeigt schematisch einen Querschnitt durch eine alternative Ausführungsform eines Feinfilters aus Figur 1 aus dem Stand der Technik. Im Unterschied zur Figur 1 weist Adsorptionskörper eine hohl-zylindrische Gestalt auf und ist mit einer axial verlaufenden Öffnung 8 versehen. Der Feinfilter 1' wird bzgl. seiner Mantelfläche allseitig angeströmt (Pfeilrichtung A). Dabei tritt das Liquid an der Einlassseite 2 ein, durchströmt die Wand 3, den Adsorptionskörper 4, die Wand 5 und verlässt den Feinfilter als Filtrat an der Filtrat-Auslassseite 6. Das Filtrat wird in Richtung der Öffnung 8 aus dem Filter ausgeleitet (Pfeilrichtung B).

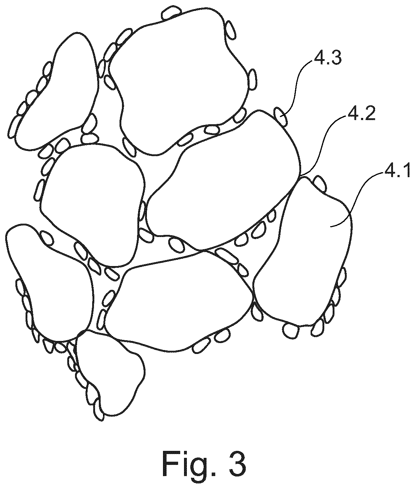

Figur 3 zeigt schematisch einen Schnitt durch ein Detail der Zusammensetzung bzw. des Aufbaus eines erfindungsgemäßen Adsorptionskörpers 4. Der Adsorptionskörper 4 weist ein Adsorptionsmittel 4.1 aus Körner aus Aktivkohle, ein Bindemittel 4.2 aus Polyethylen und eine Zirconium-Verbindung 4.3 aus Zirconiumsilicat auf. Das Adsorptionsmittel 4.1 ist mit dem Bindemittel 4.2 beschichtet. Ferner wird die Zirconium-Verbindung 4.3 vom Bindemittel 4.2 im Adsorptionskörpers 4 fixiert/gebunden.

Das Zirconiumsilicat liegt als feinkörniges Material vor, wobei Zirconiumsilicat, die Aktivkohle und das Polyethylen homogen im Adsorptionskörper 4 verteilt sind.

Das Vorhandensein von Zirconiumsilicat im Bindemittel 4.2 bzw. auf der Kohle trägt wesentlich dazu bei, die Adsorptionsfähigkeit des Adsorptionskörpers 4 zusätzlich zu erhöhen.

Der Formkörper weist nach Abkühlung Mikroporen im Adsorptionsmittel, im Bindemittel und in der Zirconium-Verbindung auf, die alle wesentlich zur Adsorption beitragen.

Die Beimengung einer Zirconiumsilicat-Verbindung im Gemisch aus Aktivkohle und Bindemittel bewirkt, dass sich der Adsorptionsgrad der Filtereinheit bei der Adsorption zum einen deutlich verbessert und die Adsorption schneller ablaufen kann. Zum anderen sind Nitrate besser adsorbierbar, wobei sich ein Nitrat-Rückgang von mehr als 3% gegenüber herkömmlichen Aktivkohlefiltern einstellt.

### Bezugszeichenliste

- 1: Filtereinheit
- 1': Filtereinheit
- 2: Einlassseite
- 3: Wand an der Einlassseite
- 4: Adsorptionskörper
- 4.1: Adsorptionsmittel
- 4.2: Bindemittel
- 4.3: Zirconium-Verbindung
- 5: Wand an der Auslassseite
- 6: Auslassseite
- 7: Gehäuse
- 8: Öffnung
- A: Pfeilrichtung
- B: Pfeilrichtung

## Patentansprüche

1. Filtereinheit (1, 1'), insbesondere zur Feinfiltration von Fluiden und Gasen, aufweisend
- mindestens eine Einlassseite (2),
- mindestens eine Auslassseite (6) und
- einen Adsorptionskörper (4) in Gestalt eines Formkörpers, der aus
a) einem Adsorptionsmittel (4.1), insbesondere aus Körnern einer Aktivkohle,
b) einem Bindemittel (4.2) und
c) einer Zirconium-Verbindung (4.3) besteht,
wobei das Adsorptionsmittel (4.1) mit dem Bindemittel (4.2) beschichtet ist und ferner die Zirconium-Verbindung (4.3) im Formkörper bindet.

2. Vorrichtung nach Anspruch 1, wobei die Zirconium-Verbindung (4.3)
- eine Zirconiumchlorid-, und/oder eine Zirconiumsilicat-Verbindung ist und
- als Pulver oder als körniges Material vorliegt.

3. Vorrichtung nach Anspruch 2, wobei die Zirconiumchlorid-, und/oder die Zirconiumsilicat-Verbindung in einem Bereich von 7,5 bis 8,4 Vol.-% des Adsorptionskörpers (4) liegt.

4. Vorrichtung nach Anspruch 1, wobei der Volumenanteil der Zirconium-Verbindung (4.3) ein Viertel bis ein Drittel des Volumenanteils des Bindemittels (4.2) beträgt.

5. Filtereinheit nach Anspruch 1, wobei der Adsorptionskörper (4) von einer einlassseitigen und einer auslassseitig anschließenden, porösen Wand (3, 5) begrenzt ist, wobei die Wände (3, 5) als mechanisches Feinfilter ausgebildet sind, wobei die Wandstärken der Wände (3, 5) jeweils im Bereich von 5% bis 10% der Stärke des Adsorptionskörpers (4) liegen.

6. Verfahren zur Herstellung einer Filtereinheit (1, 1') in Gestalt eines gas- und flüssigkeitsdurchlässigen, adsorptiven Formkörpers (4), wobei
- ein Adsorptionsmittel (4.1), insbesondere Körner aus Aktivkohle, mit einem thermoplastischen Bindemittel (4.2) beschichtet werden,
- die Körner mit einer Zirconium-Verbindung (4.3) homogen gemischt werden,
- das dabei entstehende Gemisch in eine Form eingebracht wird und in dieser komprimiert wird,
- das Gemisch in der Form bei Verhinderung von Luftzufuhr auf eine Temperatur weit über den plastischen Bereich des Bindemittels (4.2) hinaus erhitzt wird,
- die Zirconium-Verbindung im Gemisch gebunden wird und
- der so gebildeten Formkörper (4) nach Abkühlung Mikroporen im Adsorptionsmittel (4.1), im Bindemittel (4.2) und in der Zirconium-Verbindung (4.3) aufweist.

7. Verfahren gemäß Anspruch 6, wobei die Temperatur beim Erhitzen des komprimierten Gemisches zwischen 255°C und 295°C beträgt und zwischen 35 Minuten und 45 Minuten konstant gehalten wird.

8. Verfahren gemäß Anspruch 6, wobei der gebildete Formkörper (4) im flüssigen oder gasförmigen Medium abgeschreckt wird.

9. Verfahren gemäß Anspruch 8, wobei der gebildete Formkörper (4) beim Abschrecken einer Temperatur im Bereich von -185°C bis -125°C ausgesetzt wird.

10. Verfahren gemäß einem der Ansprüche 8 bis 9, wobei der Abschreckvorgang 20 Sekunden bis 180 Sekunden dauert.
